# EUROPEAN PATENT SPECIFICATION

(11) **EP 0 521 974 B1**
(45) Date of publication and mention of the grant of the patent: **13.03.1996**
(21) Application number: 91906802.3
(22) Date of filing: 25.03.1991
(51) Int. Cl.: A61K 31/43

(54) **VETERINARY TREATMENT**
BEHANDLUNG VON TIEREN
TRAITEMENT VETERINAIRE

(30) Priority: 30.03.1990 GB 9007143
(43) Date of publication of application: 13.01.1993
(73) Proprietor: BEECHAM GROUP PLC, Brentford Middlesex TW8 9EP (GB)
(72) Inventor: CRACKNELL, Victor, Charles, Dorking Road, Tadworth, Surrey KT207NT (GB)
(74) Representative: Wood, David John
(86) International application number: GB9100442
(87) International publication number: WO9115208

(56) References cited:
- EP-A- 0 010 904
- FR-A- 2 336 130
- The Veterinary Record, vol. 126, no. 13, 31 March 1990; N.J.L. Gilmour et al.: "Treatment of experimental pateurellosis in lambs with clavulanic acid and amoxycillin", p. 311

## Description

This invention relates to the treatment of pneumonic pasteurellosis in animals, particularly in lambs and sheep.

GB-B-2 005 538 describes a dry pharmaceutical composition, which comprises 20mg to 1500mg of amoxycillin trihydrate, 20mg to 500mg of potassium clavulanate and a pharmaceutically acceptable carrier, with the proviso that the weight ratio of amoxycillin trihydrate to potassium clavulanate is from 6:1 to 1:1.

Pharmaceutical compositions containing amoxycillin, clavulanic acid, and their pharmaceutically acceptable derivatives, have also been described in EP-A-0 010 904 and FR-A-2 336130.

According to the present invention there is provided the use of a formulation comprising an effective amount of amoxycillin or a veterinarily acceptable derivative thereof, clavulanic acid or a veterinarily acceptable carrier, in the manufacture of a medicament for use in the treatment of pneumonic pasteurellosis, in particular P,haemolytica.

Suitably the clavulanic acid used in the formulation is in the form of a veterinarily acceptable salt such as potassium clavulanate.

Suitably the amoxycillin used in the formulation is in the form of the trihydrate or a veterinarily acceptable ester or salt of amoxycillin such as the sodium salt.

The preferred weight ratio of amoxycillin or derivative to clavulanic acid or derivative is from 6:1 to 1:1.

Advantageously, a liquid formulation of the invention comprises 36mg/ml clavulanic acid (as potassium clavulanate) and 140mg/ml amoxycillin (as amoxycillin trihydrate).

Preferred veterinarily acceptable carriers include for example veterinarily acceptable oils such as mineral oils or fractionated coconut oil such as Miglyol 840.

Suitably the formulation is administered to the animal by intramuscular injection.

The formulation is typically prepared as an off-white suspension and is presented as a 'Ready-To-Use' form, that is the formulation is preprepared and packed in a suitable container, wherein it is ready for administration to the animal in need thereof.

The dosage rate will vary according to the size of the animal. A suitable dosage rate is generally between 2 and 25mg/kg bodyweight of the animal, for example about 10mg/kg.

Typically more than a single dose of the formulation will be required for the treatment of pneumonic pasteurellosis, suitably one dose per day for 3 to 5 days is required. In cases of severe infections prolonged treatment may be required.

Veterinary formulations for use in the present invention may be prepared by mixing the ingredients in the required proportions.

The formulation is then packaged into an appropriate container ready for administration.

The following Example illustrates the invention.

| Formulation | g/100ml |
|---|---|
| amoxycillin trihydrate | 14.0 |
| potassium clavulanate | 3.6 |
| phenol | 0.5 |
| Miglyol 840 | to 100ml |

### Biological data

1. Twenty 3-week old specific-pathogen-free Suffolk cross lambs were infected experimentally with an amoxycillin-sensitive strain of Pasteurella haemolytica, type A at a concentration of 7.43 x 10⁶ colony forming units per litre of air per minute.
2. Ten of the lambs received the formulation shown above by intramuscular injection at the recommended dose of 1ml/20Kg. of bodyweight. The treatment was repeated 24 and 48 hours later. The remaining ten lambs received no treatment.
3. Treated and untreated lambs were examined clinically for 7 days after infection when the survivors were necropsied. The results of the clinical findings are summarised in Table I.
4. One of the ten treated lambs died 6 days after infection. Seven of the ten untreated lambs died 3 (4 lambs) 4 (1 lamb) 5 (1 lamb) and 6 (1 lamb) days after infection. This difference in death rates was significant (p< 0.01 Fischer's Extract Test). In the treated group clinical abnormalities were present only in the lamb which died and that was on the day of the first treatment with the formulation.
5. Clinical abnormalities were found in 9 of the 10 untreated lambs. These clinical abnormalities and necropsy findings were similar to those expected during outbreaks of acute pastuerellosis in lambs of similar age under farm conditions. All those lambs which died had bacteriological evidence of pasteurellosis and showed either pleurisy or a subacute pneumonia consistent with Pastuerella haemolytica type A infection.

**Table I**

| Lamb No. | Pneumonia/Pleurisy Score | Recovery of P.haemolytica from: | | | |
|---|---|---|---|---|---|
| | | liver | spleen | heartblood | joint |
| 1 | 5 | + | 0 | 0 | 0 |
| 2 | 0 | 0 | 0 | 0 | 0 |
| 3 | 0 | 0 | 0 | 0 | 0 |
| 4 | 1 | 0 | 0 | 0 | 0 |
| 5 | 0 | 0 | 0 | 0 | 0 |
| 6 | 0 | 0 | 0 | 0 | + |
| 7 | 0 | 0 | 0 | 0 | 0 |
| 8 | 5 | + | + | + | + |
| 9 | 0 | 0 | 0 | 0 | 0 |
| 10 | 0 | 0 | + | 0 | + |
| 11 | 4 | + | 0 | + | 0 |
| 12 | 0 | + | + | 0 | ND |
| 13 | 2 | + | + | + | ND |
| 14 | 1 | + | + | + | + |
| 15 | 0 | + | + | + | + |
| 16 | 0 | 0 | 0 | 0 | 0 |
| 17 | 0 | 0 | + | 0 | + |
| 18 | 0 | 0 | 0 | 0 | 0 |
| 19 | 0 | + | + | 0 | 0 |
| 20 | 0 | 0 | 0 | 0 | 0 |
| ND = not done | | | | | |

It is concluded that there was a significant effect on the animals that were treated, and that the formulation has good efficacy in the treatment of pneumonic pasteurellosis in sheep.

## Claims

1. The use of a formulation comprising an effective amount of amoxycillin or a veterinarily acceptable derivative thereof, clavulanic acid or a veterinarily acceptable derivative thereof, and a veterinarily acceptable carrier, in the manufacture of a medicament for the treatment of pneumonic pasteurellosis in lambs or sheep.

2. The use according to claim 1 wherein amoxycillin is used as the trihydrate or sodium salt.

3. The use according to claim 1 or 2 wherein clavulanic acid is used as the potassium salt.

4. The use according to claim, 1, 2, or 3 wherein the carrier is an oil suitable for injection.

5. The use according to claim 1, 2, 3 or 4 wherein the amoxycillin or its derivative and the clavulanic acid or its derivative are in a weight ratio of 6:1 to 1:1.

## Patentansprüche

1. Verwendung einer Rezeptur, die eine wirksame Menge Amoxycillin oder eines veterinärmedizinisch akzeptablen Derivats hiervon, Clavulansäure oder eines veterinärmedizinisch akzeptablen Derivats hiervon und einen veterinärmedizinisch akzeptablen Träger umfaßt, bei der Herstellung eines Arzneimittels zur Verwendung bei der Behandlung von pneumonischer Pasteurellose bei Lämmern oder Schafen.

2. Verwendung nach Anspruch 1, wobei das Amoxycillin in Form des Trihydrats oder Natriumsalzes verwendet wird.

3. Verwendung nach Anspruch 1 oder 2, wobei die Clavulansäure in Form des Kaliumsalzes verwendet wird.

4. Verwendung nach Anspruch 1, 2 oder 3, wobei der Träger ein injizierbares Öl ist.

5. Verwendung nach Anspruch 1, 2, 3 oder 4, wobei das Amoxycillin oder dessen Derivat und die Clavulansäure oder deren Derivat in einem Gewichtsverhältnis von 6:1 bis 1:1 vorliegen.

## Revendications

1. Utilisation d'une formulation renfermant une quantité efficace d'amoxycilline ou d'un dérivé acceptable sur le plan vétérinaire d'un tel composé, de l'acide clavulanique ou d'un dérivé acceptable sur le plan vétérinaire d'un tel composé, et un véhicule acceptable sur le plan vétérinaire, dans la fabrication d'un médicament pour le traitement de la pasteurellose pneumonique chez l'agneau ou le mouton.

2. Utilisation selon la revendication 1, dans laquelle l'amoxycilline est utilisée sous la forme de trihydrate ou de sel sodique.

3. Utilisation selon la revendication 1 ou 2, dans laquelle l'acide clavulanique est utilisé sous forme de sel potassique.

4. Utilisation selon la revendication 1, 2 ou 3, dans laquelle le véhicule est une huile convenant à l'injection.

5. Utilisation selon la revendication 1, 2, 3 ou 4, dans laquelle l'amoxycilline ou son dérivé et l'acide clavulanique ou son dérivé sont présents dans un rapport pondéral de 6:1 à 1:1.
